# EUROPEAN PATENT APPLICATION

(11) **EP 4 603 578 A1**
(43) Date of publication of application: **20.08.2025**
(21) Application number: 24158209.7
(22) Date of filing: 16.02.2024
(51) Int. Cl.: C12N 5/078, A61K 35/17

(54) **ENGINEERED RED BLOOD CELLS AND METHOD OF PRODUCTION THEREOF**

(71) Applicant: DRK-Blutspendedienst Nord-Ost gGmbH, 01307 Dresden (DE)
(72) Inventor: Tonn, Torsten, 60323 Frankfurt am Main (DE); Kronstein-Wiedemann, Romy, 02694 Großdubrau (DE); Künzel, Stephan Reinhard, 01309 Dresden (DE); Thiel, Jessica, 01067 Dresden (DE)
(74) Representative: Kuttenkeuler, David

(57) **Abstract**

The invention relates to a method for the production of red blood cells, in particular including a method of enucleation of erythrocyte progenitor cells that were previously immortalized. The invention provides an approach for the ex-vivo generation of red blood cells for blood replacement therapies, and products associated with such use.

## Description

### FIELD OF THE INVENTION

The invention relates to a method for the production of red blood cells, in particular including a method of enucleation of erythrocyte progenitor cells that were previously immortalized. The invention provides an approach for the ex-vivo generation of red blood cells for blood replacement therapies, and products associated with such use. In addition to the generation of enucleated erythrocytes as disclosed herein, the invention further pertains to a use of nucleated erythrocyte precursors for diagnostic and therapeutic purposes as well as materials such as engineered erythrocytes of the invention immunological therapeutic strategies, such as vaccination against infections and cancer, or in treating autoimmunity.

### DESCRIPTION

Blood transfusion in anemic patients represents the most established and most common form of cell therapy. While for most patients, erythrocytes can be obtained very easily and efficiently from healthy donors, there is a small group of people displaying rare blood group constellations or multiple pre-immunizations, who cannot be supplied with compatible donor-derived blood components. Especially, when patients with very rare blood group phenotypes - that occur as founder mutation in their family - suffer from a disease with chronic transfusion requirement, such as sickle cell disease (SCD), it is often impossible to provide compatible blood components. In most cases, this is due to previous, not fully matched, transfusions of packed red blood cells (RBCs), which have resulted in multiple immunizations against common blood group antigens. Here, advances in stem cell technology have opened the door to new approaches aiming at differentiation and expansion of mature RBCs from stem cells of different tissues to provide an alternative source for packed RBCs transfusions. Recently, the term "blood pharming" has emerged to describe this innovative field of ex vivo production of blood cells. In addition to broader compatibility, cultured RBCs have several potential advantages over donor blood, such as a reduced risk of infectious disease transmission, and as the cells are all still nascent, the volume and number of transfusions could be reduced in patients requiring regular transfusions. 1, 2 In fact, the feasibility of transfusions using ex vivo generated blood stem cell derived RBCs was pioneered by the successful autologous re-infusion of 1 mL ex vivo expanded bone marrow CD34+ derived RBCs into a patient in 2011.3 In addition, the currently ongoing clinical trial "RESTORE" is studying the lifespan of the lab grown allogeneic RBCs transfused at dosages of 5-10 mL, and compares this with infusions of standard RBCs from the same donor (https://www.nhsbt.nhs.uk).

So far, ex vivo generation of erythrocytes was described from bone marrow-derived hematopoietic stem cells (HSCs) 1, 2, cord blood-derived stem cells 4-7, peripheral blood stem cells (steady phase or mobilized) 4, 8-10, embryonic stem cells (ESCs) 4, 6, 11, and induced pluripotent stem cells (iPSCs). 4, 6, 12, 13 While easily accessible, all of these potential sources have significant disadvantages. Albeit HSCs have a certain proliferation and differentiation capacity, their main limitation for routine clinical adaptation is donor dependency and their limited ex vivo expansion potential. 14 Cord blood (CB) progenitor cells yield RBCs, which possess fetal rather than adult hemoglobin. 2- To address these fallbacks of primary HSCs, also stem cell lines have been used as a source to generate red blood cells. Here, the use of iPSCs, despite unlimited expansion, is hampered by - an impaired enucleation capacity along with a lengthy differentiation process. 12, 15 Beside iPSCs, also ESCs have been used as they provide a potentially unlimited source of pluripotent stem cells for the ex vivo generation of RBCs, which on the downside is associated with both, ethical concerns and an increased risk for genomic instability and tumor formation. 16 In addition, the use of iPSCs and/or ESCs involves very lengthy and laborious in vitro protocols, since RBCs cannot be generated from iPSCs or ESCs directly and the pluripotent stem cells first need to be differentiated into HSCs. In order to shorten the process, several groups have further immortalized nucleated red blood cells obtained from iPSCs 13 and HSCs 2, 6 by introducing proto-oncogenes. In fact, Hirose et al. showed that transduction of c-myc and BCL-XL into multipotent hematopoietic progenitor cells derived from pluripotent stem cells leads to overexpression of these genes, resulting in sustained exponential self-replication of glycophorin A+ erythroblasts12. While the growth rate of such erythroblasts seems very intriguing, these cells unfortunately largely fail to further differentiate into enucleated and fully differentiated RBCs with enucleation rates of only 0.36 %.13 It has also been shown, that RBCs derived through this complex process of iPS/ES cell differentiation into HSCs, from which nucleated RBCs will be differentiated and subsequently immortalized, yield RBCs which are very different from natural occurring RBCs with regard to fragility and deformability.14

In an attempt to abbreviate the lengthy differentiation process from iPSCs and ESCs, it has recently also been suggested to immortalize primary, patient or donor derived hematopoietic CD34 cells. While CD34+ HSCs from cord blood and from bone marrow where used as a starting cell population for immortalization, immortalization of nucleated recticulocyte progenitors has only been performed on nucleated reticulocyte progenitors derived from peripheral blood mononuclear cells (PBMCs).9 However, this resulted in a heterogenic population with only around 50% of CD235a-CD71-positive cells and a limited life span of up to eight month.9

Thus, it is an object of the invention to provide novel strategies to generate red blood cells in vitro that can be applied in blood replacement therapies.

### BRIEF DESCRIPTION OF THE INVENTION

Generally, and by way of brief description, the main aspects of the present invention can be described as follows. This invention enables to generate the first human immortalized adult erythroid progenitor cell line by Dox-inducible expression of c-Myc and BCL-XL in bone marrow derived CD71-positive erythroid progenitor cells, which are termed imBMEP-A - (immortalized bone marrow erythroid progenitor adult). This is the first erythroid cell line, generated from direct immortalization of bone marrow derived reticulocyte progenitors, that produces hemoglobin in the immortalized state. The invention thus relates to the following aspects:

In **a first aspect,** the invention pertains to a method for the *in-vitro* production of immortalized genetically modifiable erythrocyte progenitor cell, comprising the steps of
- Providing a nucleated erythrocyte progenitor cell,
- Immortalizing the nucleated erythrocyte progenitor cell by an inducible transgenic expression of one or more immortalization factors,
- Optionally: propagating (expanding) the immortalized nucleated erythrocyte progenitor cell to obtain a plurality of immortalized nucleated erythrocyte progenitor cells suitable for storage.

In **a second aspect,** the invention pertains to a method for the *in-vitro* production of an erythrocyte, comprising the steps of
- Providing a nucleated erythrocyte progenitor cell,
- Immortalizing the nucleated erythrocyte progenitor cell by inducing an inducible transgenic expression of one or more immortalization factors,
- Optionally: propagating (expanding) the immortalized nucleated erythrocyte progenitor cell to obtain a plurality of immortalized nucleated erythrocyte progenitor cells;
- Stopping the induction of the inducible transgenic expression of the one or more immortalization factors and differentiating the nucleated erythrocyte progenitor cell to a reticulocyte or erythrocyte,
- Enucleating the differentiated reticulocyte or erythrocyte to obtain the final *in vitro* produced erythrocyte;
Wherein alternative to conducting steps (a) to (c), the method may comprise a step (a-c') of providing the immortalized genetically modifiable erythrocyte progenitor cell obtained by the method of claim 1, and then performing steps (d) and (e) with the so provided immortalized genetically modifiable erythrocyte progenitor cell.

In **a third aspect,** the invention pertains to an immortalized genetically modifiable erythrocyte progenitor cell, or a plurality of immortalized genetically modifiable erythrocyte progenitor cells, as obtained or obtainable by, a method of the invention.

In **a fourth aspect,** the invention pertains to an *in-vitro* produced erythrocyte, or a plurality of *in-vitro* produced erythrocytes, obtained by or obtainable by, a method of the invention.

In **a fifth aspect,** the invention pertains to a blood cell product, comprising an *in-vitro* produced erythrocyte, or a plurality of *in-vitro* produced erythrocytes according to the invention.

In **a sixth aspect,** the invention pertains to a method comprising producing a red blood cell with a method of any one of the preceding claims and formulating the red blood cell with one or more additives to form a transfusion blood composition.

In **a seventh aspect,** the invention pertains to a method of enucleating an erythrocyte or erythrocyte progenitor cell, the method comprising a step of d bringing into contact the erythrocyte or erythrocyte progenitor cell, such as a differentiated reticulocyte, with an antioxidant, an agent inhibiting the ERK1/2-SMAD2/3 signaling (such as mesalazine), and / or with one or more agent modulating hypoxy-sensitive signaling pathways such as HIF-PH pathway (such as dimethyloxalyl glycine).

In **an eight aspect,** the invention pertains to a use of a substance as enucleating agent, wherein the substance is selected from an antioxidant, an agent inhibiting the ERK1/2-SMAD2/3 signaling (such as mesalazine), and / or an agent modulating hypoxy-sensitive signaling pathways such as HIF-PH pathway (such as dimethyloxalyl glycine).

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **a first aspect,** the invention pertains to a method for the *in-vitro* production of immortalized genetically modifiable erythrocyte progenitor cell, comprising the steps of
- Providing a nucleated erythrocyte progenitor cell,
- Immortalizing the nucleated erythrocyte progenitor cell by an inducible transgenic expression of one or more immortalization factors,
- Optionally: propagating (expanding) the immortalized nucleated erythrocyte progenitor cell to obtain a plurality of immortalized nucleated erythrocyte progenitor cells suitable for storage.

In **a second aspect,** the invention pertains to a method for the *in-vitro* production of an erythrocyte, comprising the steps of
- Providing a nucleated erythrocyte progenitor cell,
- Immortalizing the nucleated erythrocyte progenitor cell by inducing an inducible transgenic expression of one or more immortalization factors,
- Optionally: propagating (expanding) the immortalized nucleated erythrocyte progenitor cell to obtain a plurality of immortalized nucleated erythrocyte progenitor cells;
- Stopping the induction of the inducible transgenic expression of the one or more immortalization factors and differentiating the nucleated erythrocyte progenitor cell to a reticulocyte or erythrocyte,
- Enucleating the differentiated reticulocyte or erythrocyte to obtain the final *in vitro* produced erythrocyte;
Wherein alternative to conducting steps (a) to (c), the method may comprise a step (a-c') of providing the immortalized genetically modifiable erythrocyte progenitor cell obtained by the method of claim 1, and then performing steps (d) and (e) with the so provided immortalized genetically modifiable erythrocyte progenitor cell.

In context of the invention an "erythrocyte progenitor cell" refers to a hematopoietic stem cell derivative within the erythroid lineage that has not yet matured into a fully differentiated erythrocyte. For example, these cells are characterized by their capacity to proliferate and differentiate into nucleated erythroid precursors, such as erythroblasts, which subsequently mature into enucleated erythrocytes (red blood cells). Erythrocyte progenitor cells are identifiable by specific surface markers, including but not limited to, CD34+, CD36+, and Glycophorin A, which differentiate them from other hematopoietic stem cells and mature erythrocytes. This definition encompasses various stages of erythroid progenitors, including erythroid burst-forming units (BFU-E) and erythroid colony-forming units (CFU-E), which are crucial for erythropoiesis, the process of red blood cell formation."

In context of the invention an erythrocyte progenitor cell can be a nucleated erythrocyte progenitor cell.

"Enucleation" refers to the process of removing the nucleus from a cell, thereby generating an enucleated cell. This procedure can be applied to various types of cells, but it is most commonly associated with the production of erythrocytes (red blood cells) in a laboratory setting. Enucleation in the prior art can be achieved through several methods, including mechanical, chemical, or genetic manipulation techniques, which facilitate the removal of the nucleus while preserving the viability and functional integrity of the remaining cell cytoplasm. The term encompasses not only the physical removal of the nucleus but also any preparatory and follow-up steps that ensure the stability, functionality, and purity of the enucleated cells for their intended application.

The present invention in particular aspects and embodiments pertain to a specific method for enucleation of a nucleated erythrocyte progenitor cell.

Preferably it is provided that the nucleated erythrocyte progenitor cell is a CD34+ hematopoietic stem cell derived *ex-vivo* from a donor sample of (mobilized) peripheral blood, or is a CD71+ bone marrow cell or umbilical cord blood; most preferably wherein the provided nucleated erythrocyte progenitor cell is a human CD71+ bone marrow cell.

In another preferred embodiment the nucleated erythrocyte progenitor cell is a direct reticulocyte progenitor cell.

In yet another embodiment, the method preferably comprises that step (b), comprises transfection or transduction of an inducible expression vector into the nucleated erythrocyte progenitor cell.

The present invention relates to an inducible expression of a transgene in order to generate an immortalized erythrocyte progenitor cell. Inducible expression is realized by introducing a nucleic acid construct or system into cells that allow for an inducible and controlled expression of the transgene of desire (such as cMyc). Such a nucleic acid construct or system includes at least one cis-acting regulatory element for directing expression of the nucleic acid sequence. Cis-acting regulatory sequences include those that direct constitutive expression of a nucleotide sequence as well as those that direct inducible expression of the nucleotide sequence only under certain conditions. Thus, for example, a promoter sequence for directing transcription of the polynucleotide sequence in the cell in a constitutive or inducible manner is included in the nucleic acid construct. In the case of mRNA, since gene expression from an RNA source does not require transcription, there is no need in a promoter sequence, or the additional sequences involved in transcription described hereinbelow. The nucleic acid construct or system (also referred to herein as an "expression vector") of some embodiments of the invention includes additional sequences which render this vector suitable for replication and integration in prokaryotes, eukaryotes, or preferably both (e.g., shuttle vectors). In addition, a typical cloning vector may also contain a transcription and/or translation initiation sequence, transcription and/or translation terminator and a polyadenylation signal. By way of example, such constructs will typically include a 5' LTR, a tRNA binding site, a packaging signal, an origin of second-strand DNA synthesis, and a 3' LTR or a portion thereof.

Eukaryotic promoters typically contain two types of recognition sequences, the TATA box and upstream promoter elements. The TATA box, located 25-30 base pairs upstream of the transcription initiation site, is thought to be involved in directing RNA polymerase to begin RNA synthesis. The other upstream promoter elements determine the rate at which transcription is initiated.

Enhancer elements can stimulate transcription up to 1,000 fold from linked homologous or heterologous promoters. Enhancers are active when placed downstream or upstream from the transcription initiation site. Many enhancer elements derived from viruses have a broad host range and are active in a variety of tissues. For example, the SV40 early gene enhancer is suitable for many cell types. Other enhancer/promoter combinations that are suitable for some embodiments of the invention include those derived from polyoma virus, human or murine cytomegalovirus (CMV), the long-term repeat from various retroviruses such as murine leukemia virus, murine or Rous sarcoma virus and HIV. See, Enhancers and Eukaryotic Expression, Cold Spring Harbor Press, Cold Spring Harbor, N.Y. 1983, which is incorporated herein by reference.

In the construction of the expression vector, the promoter is preferably positioned approximately the same distance from the heterologous transcription start site as it is from the transcription start site in its natural setting. As is known in the art, however, some variation in this distance can be accommodated without loss of promoter function.

Polyadenylation sequences can also be added to the expression vector in order to increase the efficiency of mRNA translation. Two distinct sequence elements are required for accurate and efficient polyadenylation: GU or U rich sequences located downstream from the polyadenylation site and a highly conserved sequence of six nucleotides, AAUAAA, located 11-30 nucleotides upstream. Termination and polyadenylation signals that are suitable for some embodiments of the invention include those derived from SV40.

In context of the present invention an expression vector preferably comprises at least one or more expression cassettes suitable for an inducible expression of the one or more immortalization factors; and wherein the one or more expression cassettes comprises at least in operable connection an inducible element, an erythrocyte lineage active promoter and an expressible gene sequence ending one of the one or more immortalization factors.

In further preferred embodiments, the inducible element is a DOX binding element.

In additional preferred embodiments, the expression vector is a lentiviral expression vector.

It is particularly preferred that in certain embodiments, the one or more immortalization factors are c-Myc and / or BCL-XL.

In context of the invention it is preferred that the step of differentiating the nucleated erythrocyte progenitor cell to a reticulocyte or erythrocyte comprises bringing into contact the nucleated erythrocyte progenitor cell with erythropoietin (EPO). The term "erythropoietin" and its abbreviation "EPO" refer to a protein having the amino acid sequence as shown in the Uniprot database under the accession no. P01588 (https://www.uniprot.org/uniprotkb/P01588/entry - Version of February 13, 2024) or a protein or polypeptide substantially homologous thereto, whose biological properties relate to the stimulation of red blood cell production and the stimulation of the division and differentiation of committed erythroid progenitors in the bone marrow. Recombinant erythropoietin may be prepared via expression in eukaryotic cells, for example in CHO cells, or BHK cells, or HeLa cells by recombinant DNA technology or by endogenous gene activation, i.e. the erythropoietin glycoprotein is expressed by endogenous gene activation. The term "erythropoietin" also denotes variants of the protein, in which one or more amino acid residues have been changed, deleted, or inserted, and which has comparable biological activity as the not modified protein. Such variants are known to the skilled artisan.

The method of the invention in another preferred embodiment comprises a step of genetically modifying the immortalized nucleated erythrocyte progenitor cell before differentiation, for example by genetically altering the expression of a blood group antigen, for example by gene editing.

The method of the invention in another preferred embodiment comprises enucleating in step (e) bringing into contact the differentiated reticulocyte or erythrocyte with an antioxidant, an agent inhibiting the ERK1/2-SMAD2/3 signaling (such as and preferably mesalazine), and / or with one or more agent modulating hypoxy-sensitive signaling pathways such as HIF-PH pathway (such as dimethyloxalyl glycine).

In context of the invention any compound or procedure resulting in an inhibition of ERK1/2 or SMAD2/3 signaling activity is suitable as an agent for enucleation in accordance with the invention. Specific non limiting examples ERK1/2 inhibitors are molecules capable of inhibiting the activity of ERK1/2 as determined by Western blot protein detection of phosphorylated ERK1/2 proteins. Preferably according to a particular embodiment, the ERK1/2 inhibitor is a small molecule agent. Non-limiting examples of ERK1/2 inhibitors (also known as MEK1/2 inhibitors) include PD0325901 (AXONMEDCHEM - AXON 1408), PD98059 (AXONMEDCHEM - Axon 1223), and PD 184352 (AXONMEDCHEM - AXON 1368); and/or even inhibitors of RAF (which is upstream of MEK/ERK pathway) such as Sorafenib tosylate (also known as BAY 43-9006 AXONMEDCHEM -AXON 1397) or SB 590885 (TOCRIS #2650). Others are known to the skilled artisan.

A SMAD2/3 inhibitor in context of the invention refers to a substance that blocks the SMAD2 and SMAD3 signaling pathways, which are key components of the Transforming Growth Factor-beta (TGF-β) signaling cascade. Examples of small molecular inhibitors are B-431542, LY2109761 or halofuginone.

In **a third aspect,** the invention pertains to an immortalized genetically modifiable erythrocyte progenitor cell, or a plurality of immortalized genetically modifiable erythrocyte progenitor cells, as obtained or obtainable by, a method of the invention.

In **a fourth aspect,** the invention pertains to an *in-vitro* produced erythrocyte, or a plurality of *in-vitro* produced erythrocytes, obtained by or obtainable by, a method of the invention.

In **a fifth aspect,** the invention pertains to a blood cell product, comprising an *in-vitro* produced erythrocyte, or a plurality of *in-vitro* produced erythrocytes according to the invention.

In **a sixth aspect,** the invention pertains to a method comprising producing a red blood cell with a method of any one of the preceding claims and formulating the red blood cell with one or more additives to form a transfusion blood composition.

In **a seventh aspect,** the invention pertains to a method of enucleating an erythrocyte or erythrocyte progenitor cell, the method comprising a step of d bringing into contact the erythrocyte or erythrocyte progenitor cell, such as a differentiated reticulocyte, with an antioxidant, an agent inhibiting the ERK1/2-SMAD2/3 signaling (such as mesalazine), and / or with one or more agent modulating hypoxy-sensitive signaling pathways such as HIF-PH pathway (such as dimethyloxalyl glycine).

In preferred embodiments the enucleation agent is mesalazine.

The method according to the invention is preferred, wherein the agent is brought into contact with the erythrocyte or erythrocyte progenitor cell for a time sufficient to enucleate the erythrocyte or erythrocyte progenitor cell.

In **an eight aspect,** the invention pertains to a use of a substance as enucleating agent, wherein the substance is selected from an antioxidant, an agent inhibiting the ERK1/2-SMAD2/3 signaling (such as mesalazine), and / or an agent modulating hypoxy-sensitive signaling pathways such as HIF-PH pathway (such as dimethyloxalyl glycine). Definitions of such inhibitors are provided herein above.

In addition to the generation of enucleated erythrocytes as disclosed herein, the invention further pertains to a use of nucleated erythrocyte precursors as described herein elsewhere, for diagnostic and therapeutic purposes. Furthermore, it should be understood, that the herein disclosed materials such as engineered erythrocytes are useful in immunological strategies, such as vaccination against infections and cancer, or useful in treating autoimmunity as an "adjuvant" through the expression or binding of costimulatory molecules or other binding domains.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety. A list of references is provided at the end of this specification/description.

### BRIEF DESCRIPTION OF THE FIGURES

The figures show:

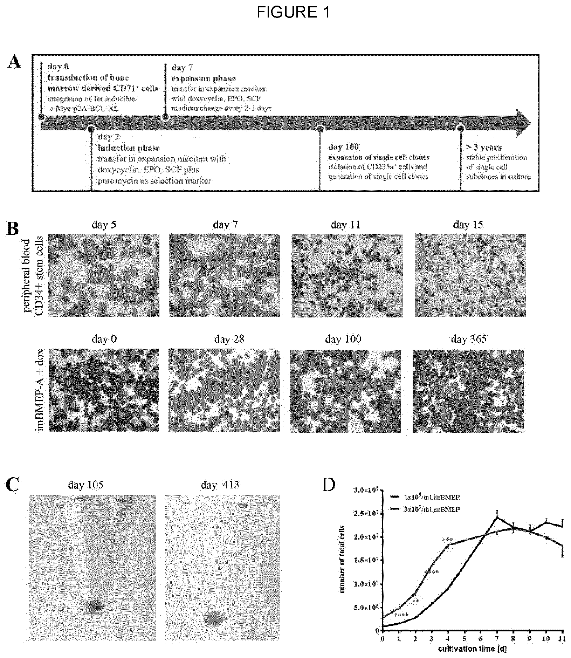

**Figure 1****:** Generation of immortalized bone marrow derived erythroid progenitor cells (imBMEP-A). Human adult bone marrow CD71-positive cells were cultured for 24 h, before transduction with the Tet-inducible c-myc-p2A-BCL-XL construct. Two days after transduction, cells were transferred to expansion medium containing doxycycline and maintained in expansion medium thereafter. (A) Scheme of experimental approach. (B) Representative cytospins illustrating similar morphology of cells proliferating in continuous culture on days 0, 28, 100 and 365 compared to peripheral blood CD34-positive HSCs on days 5, 7, 11 and 15. (C) Cell pellets of imBMEP-A cells in continuous culture on days 105 and 413. (D) Growth curve of imBMEP-A cells maintained in undifferentiated, proliferative state. Cells were seeded at either 3.0 x 105 or 1.0 x 105 cells/mL on day 0 and cells were counted regularly, with total cell number extrapolated. Doxycycline and cytokines were refreshed every 3 to 4 days to maintain cells in undifferentiated state. n = 3. Data are presented as mean ± SEM. Tests were performed two-sided. **P < 0.01; ***P < 0.001, ****P < 0.0001 (student t test).

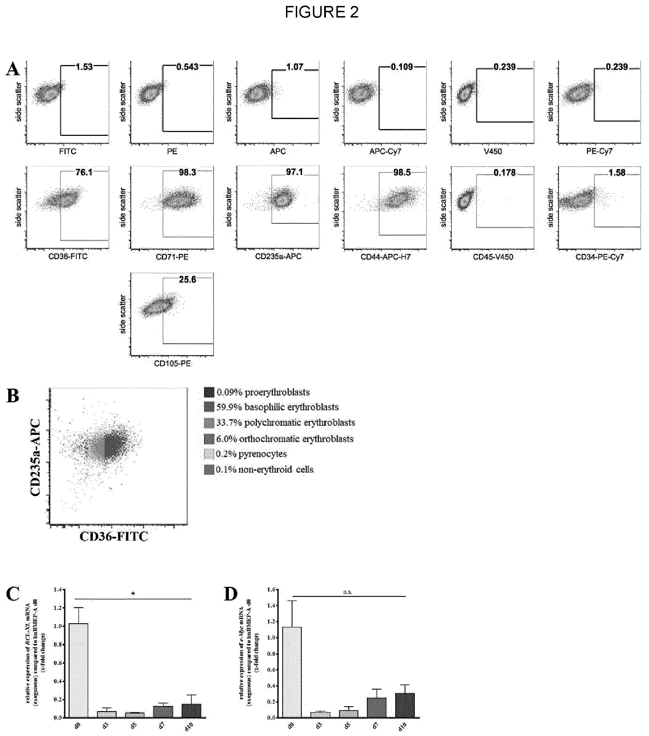

**Figure 2****:** ImBMEP-A cells show characteristic markers for basophilic and polychromatic erythroblasts. (A) Flow cytometric analysis of imBMEP cells. Cells were positive for different erythroid surface marker (CD36, CD71, CD44, CD105, CD235a) and negative for the stem cell markers CD34 and CD45. (B) Percentage of different erythroid cell subtypes in the heterogeneous imBMEP-A cell line. (C-D) Real-time PCR expression analysis of exogenous BCL-XL (C) and exogenous c-myc (D) in imBMEP-A cells in the presence and absence of doxycycline (n = 3 independent differentiation experiments). Relative fold changes in expression (normalized to GAPDH) were calculated by the □□CT method and values are expressed as 2-□□CT to the mean values of undifferentiated control (day 0). Data are presented as mean ± SEM. Tests were performed two-sided. *p < 0.05 (student t test).

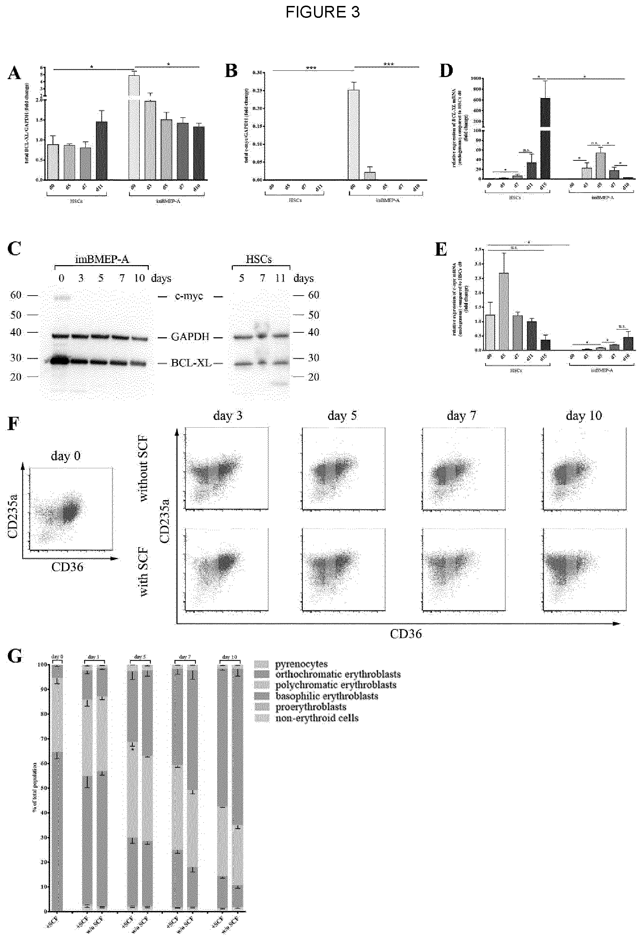

**Figure 3****:** ImBMEP-A cells are capable for terminal differentiation. (A-B) Quantification of total BCL-XL (A) and c-myc (B) protein expression by western blot analysis during ex vivo erythropoiesis of primary HSCs and imBMEP-A cells. Expression was normalized to GAPDH protein expression as endogeneous loading control (fold change). (C) Western blot analysis from primary HSCs and imBMEP-A cells during erythropoiesis using antibodies against BCL-XL, c-myc and GAPDH as loading control. One of three representative experiments is shown. (D-E) Real-time PCR expression analysis of endogenous BCL-XL (C) and endogenous c-myc (D) in imBMEP-A cells in the presence and absence of doxycycline (n = 3 independent differentiation experiments) and primary HSCs. Relative fold changes in expression (normalized to GAPDH) were calculated by the □□CT method and values are expressed as 2-□□CT to the mean values of undifferentiated control (day 0). (F) Flow cytometric analysis of imBMEP-A cells during erythropoiesis in the presence and absence of SCF. Cells were double stained with FITC-labelled anti-CD36 and APC-labelled anti-CD235a antibodies. (G) Quantitative analysis of flow cytometric analysis. Distribution of erythroid differentiation stages were determined by the CD36-CD235a expression pattern of the cell population. n = 4. Data are presented as mean ± SEM. Tests were performed two-sided. *P < 0.05 (Mann-Whitney U test). (A-B, D-E) n = 3. Data are presented as mean ± SEM. Tests were performed two-sided. *P < 0.05 (student t test).

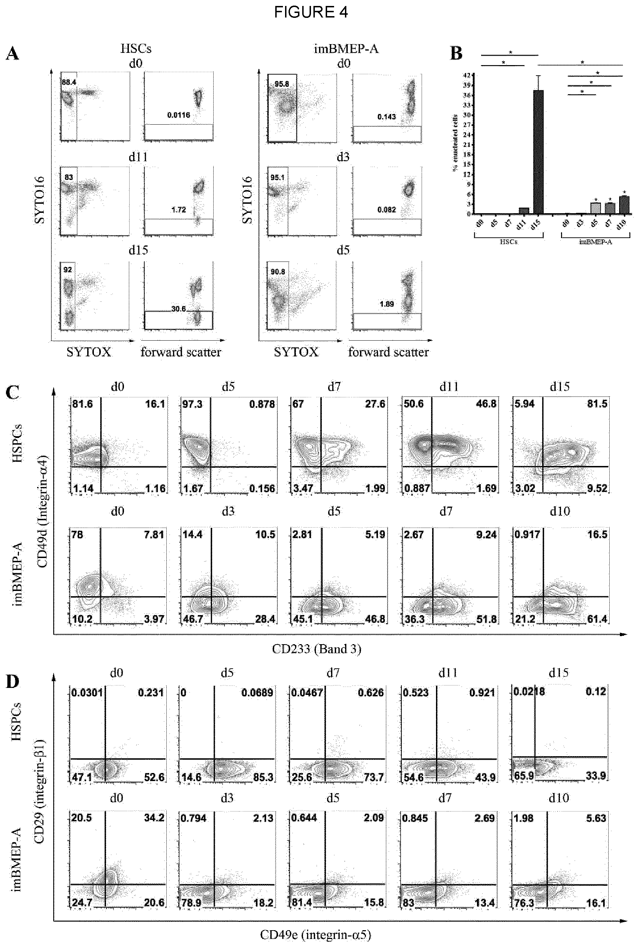

**Figure 4****:** ImBMEP-A cells are able to differentiate into enucleated red blood cells. (A) Evaluation of enucleated red blood cells derived from differentiated primary HSCs and imBMEP-A cells, determined via SYTO16-SYTOX flow cytometric analysis. One of four representative experiments is shown. (B) Quantitative analysis of enucleation rate. n = 4. Data are presented as mean ± SEM. Tests were performed two-sided. *P < 0.05 (Mann-Whitney U test). (C-D) Flow cytometric analysis of imBMEP-A cells and primary HSCs during erythropoiesis. Cells were dual stained by incubation with antibodies to α4 integrin and band 3 (C) as well as □1-integrin and α5-integrin (D).

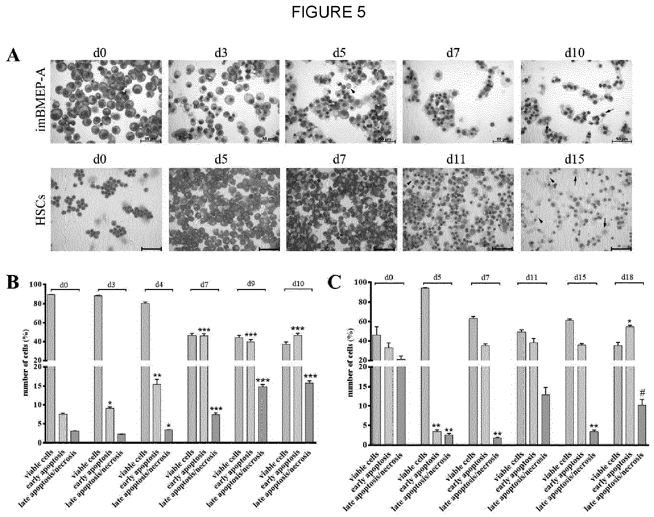

**Figure 5****:** ImBMEP-A cells showed increased apoptosis compared to HSCs during erythroid differentiation. (A) Morphological analysis of primary HSCs and imBMEP-A cells during ex vivo erythropoiesis. Cells were stained with Giemsa solution (bar = 50µm). Arrows indicate dead cells and arrow heads indicate enucleated mature RBCs. (B) Apoptosis assay of imBMEP-A cells during erythroid differentiation. (C) Apoptosis assay of primary HSCs during erythroid differentiation. (B-C) n = 3. Data are presented as mean ± SEM. Tests were performed two-sided. *P < 0.05, **P < 0.01, ***P < 0.001, # significance between stem cells and imBMEP-A cells (student t test).

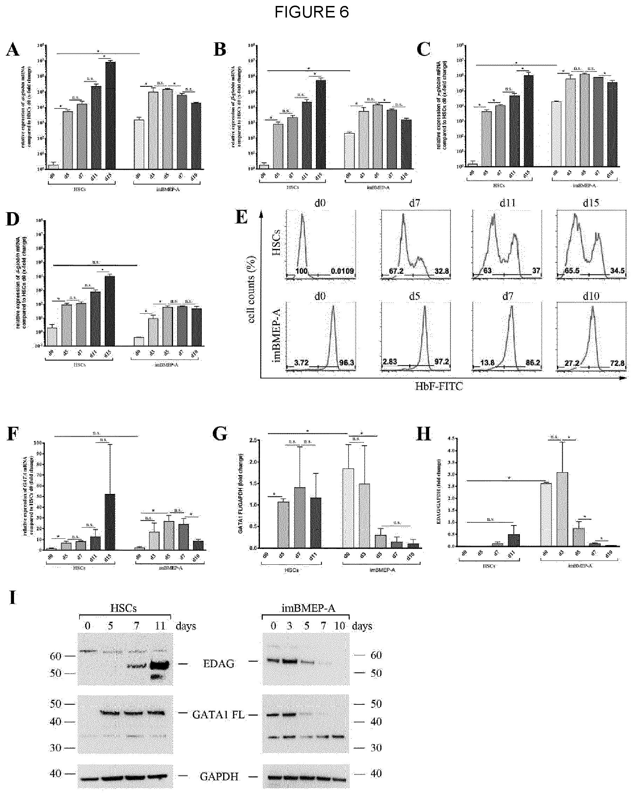

**Figure 6****:** imBMEP-A cells express globin chains from both fetal and adult hemoglobin. (A-D) Real-time PCR expression analysis of □-globin (A), □-globin (B), □-globin (C), and □-globin mRNA (D) in imBMEP-A cells in the presence and absence of doxycycline (n = 3 independent differentiation experiments) and primary HSCs. (E) Flow cytometric analysis of fetal Hb using FMH Quikquant^{™} kit in imBMEP-A cells and primary HSCs during erythroid differentiation. (F) Real-time PCR expression analysis of GATA-1 mRNA (D) in imBMEP-A cells in the presence and absence of doxycycline and primary HSCs. (G-H) Quantification of full length GATA-1 (G) and EDAG (H) protein expression by western blot analysis during ex vivo erythropoiesis of primary HSCs and imBMEP-A cells. Expression was normalized to GAPDH protein expression as endogenous loading control (fold change). (I) Western blot analysis from primary HSCs and imBMEP-A cells during erythropoiesis using antibodies against GATA-1, EDAG and GAPDH as loading control. One of three representative experiments is shown. (A-D, F) Relative fold changes in expression (normalized to GAPDH) were calculated by the □□CT method and values are expressed as 2-□□CT to the mean values of primary HSCs (day 0). (A-D, F, G-H) n = 3 independent differentiation experiments. Data are presented as mean ± SEM. Tests were performed two-sided. *P < 0.05, n.s. not significant (student t test).

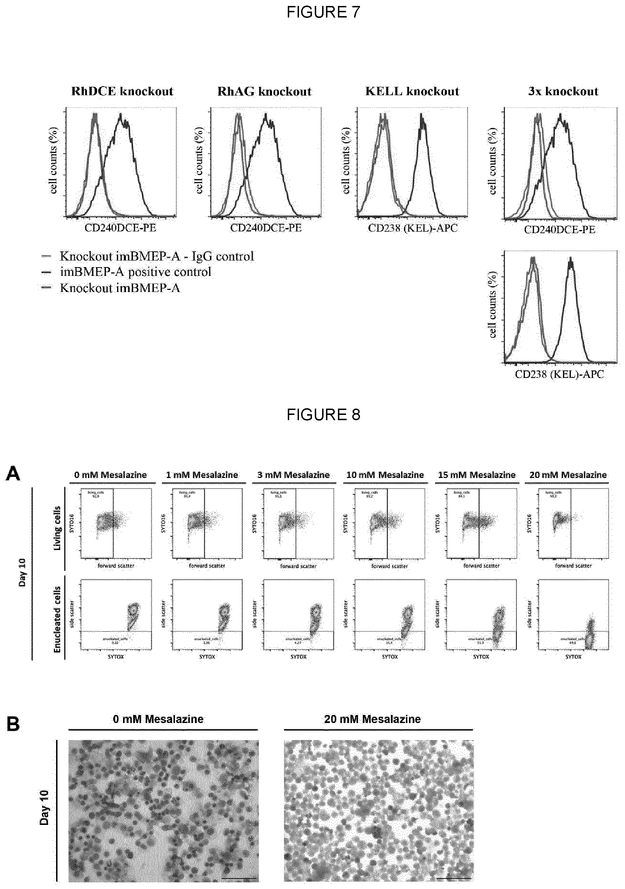

**Figure 7****:** imBMEP-A cells are amenable to genetic manipulation. Flow cytometric confirmation of individual blood group knockouts in imBMEP-A cells. imBMEP-A blood group knockout lines were created using Cas9 mRNA and synthetic sgRNAs gene editing and single cell sorted into clonal sub-lines. Expression levels of targeted blood group antigens in knockout lines overlay with IgG controls indicating complete protein knockouts. Cells were stained with antibodies against either PE-labelled CD240DCE or APC-labelled CD238 (KEL). One of five representative single-cell clones of each individual knockout is shown.
Figure 8: Mesalazine treatment of imBMEP-A cells leads to drastical increase of **terminal erythroid differentiation.** Cells were cultivated in the absence of doxycyclin and stimulated with different concentrations of mesalazine for 10 days. (**A**) Evaluation of enucleated red blood cells derived from imBMEP-A cells, determined via SYTO16-SYTOX flow cytometric analysis. (**B**) Morphological analysis of imBMEP-A cells during ex vivo erythropoiesis in the absence or presence of 20 mM mesalazine. Cells were stained with Giemsa solution (bar = 50µm).

### EXAM PLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Generation of stable erythroid progenitor cell lines from human CD71-positive cells with inducible c-myc-p2A-BCL-XL expression

Isolated CD71-positive cells from human bone marrow (BM) were transduced with c-myc-p2A-BCL-XL using an antibiotic-inducible lentiviral vector system. 48 h after transduction, the induction phase was initiated by adding doxycycline and puromycin to select transduced cells. At day 100, CD235a-positive cells were isolated from the heterogenous immortalized cell population and single-cell clones were generated with proven monoclonality by using the single-cell-printing technology (SCP).¹⁷ **Figure 1A** visualizes the outline of the protocol. Based on the extent of proliferation rate and expression of erythroid markers, as well as hemoglobin formation, we selected clone 34 (hereinafter referred to as imBMEP-A) for all further analyses. The imBMEP-A cells displayed continuous proliferation and were frozen after 170 days - well beyond the Hayflick limit ¹⁸. Additionally, samples were frozen at regular intervals throughout this period and reestablished efficiently in culture following freezing and thawing. The mean doubling time of the cells after one year in continuous culture was 49.1 h ± 6.5 h. Morphological analysis of the immortalized cells showed that they are nucleated cells with largely basophilic erythroblast character as compared to peripheral blood CD34+ hematopoietic stem cells, which were mostly enucleated after 15 days (**Fig. 1B**). There were no changes in morphology over time. Furthermore, pelleted erythroid cells from imBMEP-A line showed the typical red color, which indicates hemoglobin production (**Fig. 1C**). The expansion profile of imBMEP-A after 200 days in culture shows an exponential growth over 7 days until a plateau is finally reached (**Fig. 1D**). Transduction with other oncogenes, such as SV40 LargeT antigen, LhX-2, Bmi-1 or c-myc and BCL-XL alone did not result in continuous proliferation and cells died after a maximum of 40 days (data not shown).

### Example 2: Characterization of the imBMEP-A line

To confirm the basophilic erythroblast character of the imBMEP-A line we performed flow cytometric analysis for lineage-specific surface markers. ImBMEP-A cells highly expressed erythroid markers such as CD71, CD36, CD44, and CD235a, and were negative for the hematopoietic stem cell markers CD34 and CD45 (**Fig. 2A**). Furthermore, we subdivided the cells into different subpopulations according to the expression pattern of specific surface markers such as CD36 and CD235a.¹⁹ The preferred cell type of the imBMEP-A line corresponded to basophilic erythroblasts. A small fraction showed characteristic expression patterns for polychromatic erythroblasts (**Fig 2B**).

### Example 3: ImBMEP-A cells are capable of terminal differentiation

Removal of doxycycline from the culture medium led to silencing of exogenous BCL-XL and c-myc, measured at both the mRNA (**Fig. 2C****, D**) and protein levels (Fig. 3A-C). Interestingly, during normal erythropoiesis of HSCs, endogenous *BCL-XL* was upregulated more than 600-fold until day 15 of erythroid differentiation (**Fig. 3D**). In contrast, endogenous *BCL-XL* expression was upregulated by only one-tenth of the physiological level observed in HSCs, once the exogenous *BCL-XL* gene was silenced in imBMEP-A cells. The highest levels of endogenous *BCL-XL* were observed at day 5 of erythroid differentiation and afterwards it was downregulated. An opposite effect was observed for the expression of endogenous *c-myc* mRNA (**Fig. 3E**). During physiological erythropoiesis of HSCs *c-myc* is downregulated, whereas imBMEP-A cells showed a slightly but significant upregulation of *c-myc* mRNA.

Furthermore, the number of orthochromatic erythroblasts increased dramatically over differentiation time. The absence of SCF during differentiation yielded significantly more orthochromatic erythroblasts from day 5 onward compared to cell cultures with SCF supplementation (**Fig. 3F****, G**). Orthochromatic erythroblasts were highly positive for CD235a, but negative for CD36.¹⁹

The enucleation process was monitored using two different DNA-staining dyes, SYTO16 and SYTOX blue, which are cell-permeable and cell-impermeable fluorescent dyes, respectively ²⁰ (**Fig. 4A****, B**). Whereas HSCs yielded ~ 40 % enucleated cells after 15 days of erythroid differentiation culture, the same conditions resulted in only a small amount of enucleated reticulocytes in the imBMEP-A line (~ 5 % at day 10 of erythroid differentiation).

To assess differentiation in more detail, the expression of Band 3 (CD233) vs D4-integrin and α5-integrin vs β1-integrin was analyzed using dual staining. During erythroid differentiation, the expression of Band 3 protein increased, whereas that of α4-integrin expression strongly decreased. In contrast, the expression of α4-integrin in HSCs was only slightly decreased at the end of differentiation (day 10) (**Fig. 4C**). A similar pattern was seen when cells were stained with anti-α5-integrin (CD49e) (**Fig. 4D**), whereas β1-integrin (CD29) was not detected in both HSCs and imBMEP-A cells during erythroid differentiation. However, undifferentiated imBMEP-A cells still expressed β1-integrin in ~50 % of cells.

### Example 4: ImBMEP-A cells showed increased apoptosis compared to HSCs during erythroid differentiation

Morphologically, imBMEP-A cells showed decreased cell size, more condensed nuclei, and a lighter cytoplasm during differentiation, which is comparable to the differentiation process of HSCs (**Fig. 5A**). At day 7 of differentiation, imBMEP-A cells corresponded to orthochromatic erythroblasts, resembling day 11 of HSC differentiation. In HSC-derived differentiation cultures, we observed many enucleated reticulocytes at day 15 and day 18, whereas differentiation of imBMEP-A led to only very low numbers of enucleated cells (arrowheads in **Fig. 5A**). However, increased cell death was detectable (arrows). Thus, apoptosis was subsequently evaluated by Annexin V and PI staining in both differentiated imBMEP-A cells (**Fig. 5B**) and HSCs (**Fig. 5C**). There was a strong increase of early apoptotic cells from day 3 and late apoptotic/necrotic cells from day 5, respectively. Due to the thawing process, HSC cultures contained a large population of early apoptotic cells, which decreased during the first three days until it finally normalized. However, the population of late apoptotic cells was significantly lower compared to imBMEP-A cells and did not increase significantly throughout the course of differentiation.

### Example 5: imBMEP-A cells express globin chains of both fetal and adult hemoglobin

We next examined differential globin chain expression in imBMEP-A cells and HSCs at the mRNA level (Fig. 6A-D). In contrast to HSCs, inBMEP-A cells already expressed α-, β-, γ- and δ-globin at day 0 (undifferentiated state). The respective levels of globin chains expression was similar to that of HSCs of erythroid differentiation at day 5, except for the □-globin, which was similar to the expression in HSCs at day 0. Flow cytometry revealed further differences in hemoglobin type composition between HSCs and imBMEP-Acells. Whereas the majority of HSCs produce adult hemoglobin (FITC-negative section) after induction of hemoglobinization at day 7 - 15, imBMEP-A cells show a predominant proportion of fetal hemoglobin (FITC-positive section) throughout the entire differentiation period. Instead of a clear switch from fetal to adult hemoglobin type, only a slight shift is detectable after 10 days of differentiation (**Fig. 6E**).

Regarding TF *GATA1*, which plays a central role in erythropoiesis ²¹, there was a slight increase in mRNA expression until day 5 which dropped to the original level on day 10 (**Fig. 6F**). In HSCs, mRNA expression of *GATA1* was continuously upregulated during erythropoiesis. Similar, protein levels of GATA1 increased steadily during erythropoiesis in HSCs, unlike in imBMEP-A cells, where GATA1 expression increased until day 5 and then decreased again (**Fig. 6G**). Another important factor of erythroid differentiation, the erythroid differentiation-associated gene (EDAG) ²² was upregulated during normal erythropoiesis (**Fig. 6H****, I**), but markedly downregulated during differentiation of imBMEP-A cells.

### Example 7: imBMEP-A cells are amenable to genetic manipulation

As it is our aim to produce universal compatible RBCs, we next investigated, whether the cells are amenable to genetic manipulation i.e. to the knock out of different blood group antigens. Since the imBMEP-A cell line was derived from a donor of Rh type D+C-c+E+e-, both RhD and RhCE required removal. Thus, single sgRNAs targeting the RH-associated glycoprotein (RHAG) were designed. Mutations in RHAG result in Rhₙᵤₗₗ erythrocytes, demonstrating that RHAG is essential for stable expression of Rh.^{23, 24} The Rhₙᵤₗₗ phenotype is associated with mild compensated anemia.²⁵ Therefore, in parallel, we designed single sgRNAs targeting only RHD/CE rather than completely removing RhAG. Furthermore, we knocked out *KEL2* expression, which corresponds to the KEL negative phenotype. Afterwards, single-cell clones were generated and analyzed for absence of CD240DCE and CD238 expression. We provided two targeted sgRNAs and generated single-cell clones from those cells in which the knock out with the corresponding sgRNA was most successful. **Fig. 7** shows the complete absence of expression of proteins targeted by CRISPR gRNAs in knockout imBMEP-A cells compared with unedited imBMEP-A cells, as measured by flow cytometry. In parallel, 3x knockout was performed using single gRNAs to target RhAG along with single gRNAs to target KEL2.

### Example 8: Enucleation method

Currently, the highest enucleation rate achieved during differentiation of immortalized erythroid cells is approximately 30% (doi: 10.1016/j.omtm.2021.06.002). However, a much higher rate of at least 70-80% is required for successful practicable clinical application. Therefore, we performed optimization of culture conditions and tested different substances that influence different pathways involved in enucleation process. We treated imBMEP-A cells with different concentrations of mesalazine in the absence of doxycycline and measured the enucleation rate at day 10 of differentiation. The enucleation process was monitored using two different nucleic acid dyes. While the green fluorescent SYTO16 is membrane-permeable, SYTOX Blue only penetrates dead cells with compromised plasma membranes (doi:10.1038/nature03964). Mesalazine treatment of imBMEP-A cells leads to drastical increase of terminal erythroid differentiation (Fig. 8A). Giemsa staining of cells treated with 20mM mesalazine showed more than 80% enucleated reticulocytes (Fig. 8B).

### REFERENCES

The references are:
1. Daniels DE, Ferguson DCJ, Griffiths RE, Trakarnsanga K, Cogan N, Maclnnes KA, et al. Reproducible immortalization of erythroblasts from multiple stem cell sources provides approach for sustainable RBC therapeutics. Mol Ther Methods Clin Dev 2021 Sep 10; 22: 26-39.
2. Trakarnsanga K, Griffiths RE, Wilson MC, Blair A, Satchwell TJ, Meinders M, et al. An immortalized adult human erythroid line facilitates sustainable and scalable generation of functional red cells. Nature communications 2017 Mar 14; 8: 14750.
3. Giarratana MC, Rouard H, Dumont A, Kiger L, Safeukui I, Le Pennec PY, et al. Proof of principle for transfusion of in vitro-generated red blood cells. Blood 2011 Nov 10; 118(19): 5071-5079.
4. Cervellera CF, Mazziotta C, Di Mauro G, laquinta MR, Mazzoni E, Torreggiani E, et al. Immortalized erythroid cells as a novel frontier for in vitro blood production: current approaches and potential clinical application. Stem cell research & therapy 2023 May 24; 14(1): 139.
5. Huang X, Shah S, Wang J, Ye Z, Dowey SN, Tsang KM, et al. Extensive ex vivo expansion of functional human erythroid precursors established from umbilical cord blood cells by defined factors. Molecular therapy: the journal of the American Society of Gene Therapy 2014 Feb; 22(2): 451-463.
6. Kurita R, Suda N, Sudo K, Miharada K, Hiroyama T, Miyoshi H, et al. Establishment of immortalized human erythroid progenitor cell lines able to produce enucleated red blood cells. PloS one 2013; 8(3): e59890.
7. Zhang Y, Wang C, Wang L, Shen B, Guan X, Tian J, et al. Large-Scale Ex Vivo Generation of Human Red Blood Cells from Cord Blood CD34(+) Cells. Stem cells translational medicine 2017 Aug; 6(8): 1698-1709.
8. Christaki EE, Politou M, Antonelou M, Athanasopoulos A, Simantirakis E, Seghatchian J, et al. Ex vivo generation of transfusable red blood cells from various stem cell sources: A concise revisit of where we are now. Transfus Apher Sci 2019 Feb; 58(1): 108-112.
9. Lee E, Lim ZR, Chen HY, Yang BX, Lam AT, Chen AK, et al. Defined Serum-Free Medium for Bioreactor Culture of an Immortalized Human Erythroblast Cell Line. Biotechnol J 2018 Apr; 13(4): e1700567.
10. Scully EJ, Shabani E, Rangel GW, Gruring C, Kanjee U, Clark MA, et al. Generation of an immortalized erythroid progenitor cell line from peripheral blood: A model system for the functional analysis of Plasmodium spp. invasion. Am J Hematol 2019 Sep; 94(9): 963-974.
11. Rouzbeh S, Kobari L, Cambot M, Mazurier C, Hebert N, Faussat AM, et al. Molecular signature of erythroblast enucleation in human embryonic stem cells. Stem Cells 2015 Aug; 33(8): 2431-2441.
12. Dorn I, Klich K, Arauzo-Bravo MJ, Radstaak M, Santourlidis S, Ghanjati F, et al. Erythroid differentiation of human induced pluripotent stem cells is independent of donor cell type of origin. Haematologica 2015 Jan; 100(1): 32-41.
13. Hirose S, Takayama N, Nakamura S, Nagasawa K, Ochi K, Hirata S, et al. Immortalization of erythroblasts by c-MYC and BCL-XL enables large-scale erythrocyte production from human pluripotent stem cells. Stem cell reports 2013; 1(6): 499-508.
14. Lanza F, Seghatchian J. Trends and targets of various types of stem cell derived transfusable RBC substitution therapy: Obstacles that need to be converted to opportunity. Transfus Apher Sci 2020 Oct; 59(5): 102941.
15. Trakarnsanga K, Wilson MC, Griffiths RE, Toye AM, Carpenter L, Heesom KJ, et al. Qualitative and quantitative comparison of the proteome of erythroid cells differentiated from human iPSCs and adult erythroid cells by multiplex TMT labelling and nanoLC-MS/MS. PloS one 2014; 9(7): e100874.
16. Cherry AB, Daley GQ. Reprogramming cellular identity for regenerative medicine. Cell 2012 Mar 16; 148(6): 1110-1122.
17. Gross A, Schondube J, Niekrawitz S, Streule W, Riegger L, Zengerle R, et al. Single-cell printer: automated, on demand, and label free. J Lab Autom 2013 Dec; 18(6): 504-518.
18. Shay JW, Wright WE. Hayflick, his limit, and cellular ageing. Nat Rev Mol Cell Biol 2000 Oct; 1(1): 72-76.
19. Tirelli V, Ghinassi B, Migliaccio AR, Whitsett C, Masiello F, Sanchez M, et al. Phenotypic definition of the progenitor cells with erythroid differentiation potential present in human adult blood. Stem Cells Int 2011; 2011: 602483.
20. Yoshida H, Kawane K, Koike M, Mori Y, Uchiyama Y, Nagata S. Phosphatidylserine-dependent engulfment by macrophages of nuclei from erythroid precursor cells. Nature 2005 Sep 29; 437(7059): 754-758.
21. Cantor AB, Orkin SH. Transcriptional regulation of erythropoiesis: an affair involving multiple partners. Oncogene 2002 May 13; 21(21): 3368-3376.
22. Dong XM, Zhao K, Zheng WW, Xu CW, Zhang MJ, Yin RH, et al. EDAG mediates Hsp70 nuclear localization in erythroblasts and rescues dyserythropoiesis in myelodysplastic syndrome. FASEB J 2020 Jun; 34(6): 8416-8427.
23. Hawksworth J, Satchwell TJ, Meinders M, Daniels DE, Regan F, Thornton NM, et al. Enhancement of red blood cell transfusion compatibility using CRISPR-mediated erythroblast gene editing. EMBO molecular medicine 2018 Jun; 10(6).
24. Huang CH. The human Rh50 glycoprotein gene. Structural organization and associated splicing defect resulting in Rh(null) disease. J Biol Chem 1998 Jan 23; 273(4): 2207-2213.
25. Sturgeon P. Hematological observations on the anemia associated with blood type Rhnull. Blood 1970 Sep; 36(3): 310-320.
26. Mei Y, Liu Y, Ji P. Understanding terminal erythropoiesis: An update on chromatin condensation, enucleation, and reticulocyte maturation. Blood reviews 2020 Mar; 46: 100740.
27. Menon V, Ghaffari S. Erythroid enucleation: a gateway into a "bloody" world. Experimental hematology 2021 Mar; 95: 13-22.
28. Kurita R, Funato K, Abe T, Watanabe Y, Shiba M, Tadokoro K, et al. Establishment and characterization of immortalized erythroid progenitor cell lines derived from a common cell source. Experimental hematology 2019 Jan; 69: 11-16.
29. Soboleva S, Kurita R, Kajitani N, Akerstrand H, Miharada K. Establishment of an immortalized human erythroid cell line sustaining differentiation potential without inducible gene expression system. Hum Cell 2022 Jan; 35(1): 408-417.
30. Hafid-Medheb K, Poindessous-Jazat V, Augery-Bourget Y, Hanania N, Robert-Lezenes J. Bcl-XL induction during terminal differentiation of friend erythroleukaemia cells correlates with delay of apoptosis and loss of proliferative capacity but not with haemoglobinization. Cell Death Differ 1999 Feb; 6(2): 166-174.
31. You X, Liu F, Zhang T, Lv N, Liu Q, Shan C, et al. Hepatitis B virus X protein upregulates Lin28A/Lin28B through Sp-1/c-Myc to enhance the proliferation of hepatoma cells. Oncogene 2014 Jan 23; 33(4): 449-460.
32. Lee YT, de Vasconcellos JF, Yuan J, Byrnes C, Noh SJ, Meier ER, et al. LIN28B-mediated expression of fetal hemoglobin and production of fetal-like erythrocytes from adult human erythroblasts ex vivo. Blood 2013 Aug 8; 122(6): 1034-1041.

## Claims

1. **A method for the *in-vitro* production of immortalized genetically modifiable erythrocyte progenitor cell,** comprising the steps of
(a) Providing a nucleated erythrocyte progenitor cell,
(b) Immortalizing the nucleated erythrocyte progenitor cell by an inducible transgenic expression of one or more immortalization factors,
(c) Optionally: propagating (expanding) the immortalized nucleated erythrocyte progenitor cell to obtain a plurality of immortalized nucleated erythrocyte progenitor cells suitable for storage.

2. **A method for the *in-vitro* production of an erythrocyte,** comprising the steps of
(a) Providing a nucleated erythrocyte progenitor cell,
(b) Immortalizing the nucleated erythrocyte progenitor cell by inducing an inducible transgenic expression of one or more immortalization factors,
(c) Optionally: propagating (expanding) the immortalized nucleated erythrocyte progenitor cell to obtain a plurality of immortalized nucleated erythrocyte progenitor cells;
(d) Stopping the induction of the inducible transgenic expression of the one or more immortalization factors and differentiating the nucleated erythrocyte progenitor cell to a reticulocyte or erythrocyte,
(e) Enucleating the differentiated reticulocyte or erythrocyte to obtain the final *in vitro* produced erythrocyte;
Wherein alternative to conducting steps (a) to (c), the method may comprise a step (a-c') of providing the immortalized genetically modifiable erythrocyte progenitor cell obtained by the method of claim 1, and then performing steps (d) and (e) with the so provided immortalized genetically modifiable erythrocyte progenitor cell.

3. The method of claim 1 or 2, wherein the provided nucleated erythrocyte progenitor cell is a CD34+ hematopoietic stem cell derived *ex-vivo* from a donor sample of (mobilized) peripheral blood, or is a CD71+ bone marrow cell or umbilical cord blood; most preferably wherein the provided nucleated erythrocyte progenitor cell is a human CD71+ bone marrow cell.

4. The method of any one of claims 1 to 3, wherein the nucleated erythrocyte progenitor cell is a direct reticulocyte progenitor cell.

5. The method of claim 4, wherein the expression vector comprises at least one or more expression cassettes suitable for an inducible expression of the one or more immortalization factors; and wherein the one or more expression cassettes comprises at least in operable connection an inducible element, an erythrocyte lineage active promoter and an expressible gene sequence ending one of the one or more immortalization factors.

6. The method of any one of claims 1 to 5, wherein the one or more immortalization factors are c-Myc and / or BLC-XL.

7. The method of any one of claims 2, and 3 to 6 when referring to claim 2, wherein the step of differentiating the nucleated erythrocyte progenitor cell to a reticulocyte or erythrocyte comprises bringing into contact the nucleated erythrocyte progenitor cell with erythropoietin (EPO).

8. The method of any one of the preceding claims, comprising a step of genetically modifying the immortalized nucleated erythrocyte progenitor cell before differentiation, for example by genetically altering the expression of a blood group antigen, for example by gene editing.

9. The method of any one of claims 2, and 3 to 8 when referring to claim 2, wherein enucleating in step (e) comprises bringing into contact the differentiated reticulocyte or erythrocyte with an antioxidant, an agent inhibiting the ERK1/2-SMAD2/3 signaling (such as mesalazine), and / or with one or more agent modulating hypoxy-sensitive signaling pathways such as HIF-PH pathway (such as dimethyloxalyl glycine).

10. **An immortalized genetically modifiable erythrocyte progenitor cell, or a plurality of immortalized genetically modifiable erythrocyte progenitor cells,** as obtained or obtainable by, a method of any one of claims 1 to 9.

11. **An *in-vitro* produced erythrocyte, or a plurality of *in-vitro* produced erythrocytes,** obtained by or obtainable by, a method of any one of claims 2 or 3 to 9 when referring to claim 2.

12. **A blood cell product,** comprising an *in-vitro* produced erythrocyte, or a plurality of *in-vitro* produced erythrocytes according to claim 11.

13. **A method of producing transfusion blood compositions,** the method comprising producing a red blood cell with a method of any one of the preceding claims and formulating the red blood cell with one or more additives to form a transfusion blood composition.

14. **A method of enucleating an erythrocyte or an (nucleated) erythrocyte progenitor cell,** the method comprising a step of d bringing into contact the erythrocyte or erythrocyte progenitor cell, such as a differentiated reticulocyte, with an antioxidant, an agent inhibiting the ERK1/2-SMAD2/3 signaling (such as mesalazine), and / or with one or more agent modulating hypoxy-sensitive signaling pathways such as HIF-PH pathway (such as dimethyloxalyl glycine).

15. **A use of a substance as enucleating agent,** wherein the substance is selected from an antioxidant, an agent inhibiting the ERK1/2-SMAD2/3 signaling (such as mesalazine), and / or an agent modulating hypoxy-sensitive signaling pathways such as HIF-PH pathway (such as dimethyloxalyl glycine).
